Europäisches Patentamt

European Patent Office

Office européen des brevets

(19)

(11) Veröffentlichungsnummer : **0 121 672**
**B1**

(12) **EUROPÄISCHE PATENTSCHRIFT**

(45) Veröffentlichungstag der Patentschrift :
12.11.86

(51) Int. Cl.⁴ : **C 07 C 37/60, C 07 C 39/08**

(21) Anmeldenummer : 84101172.9

(22) Anmeldetag : 06.02.84

(54) Verfahren zur Herstellung von Brenzcatechin und Hydrochinon.

(30) Priorität : 11.03.83 DE 3308737

(43) Veröffentlichungstag der Anmeldung :
17.10.84 Patentblatt 84/42

(45) Bekanntmachung des Hinweises auf die Patenterteilung : 12.11.86 Patentblatt 86/46

(84) Benannte Vertragsstaaten :
AT BE CH DE FR GB IT LI LU NL SE

(56) Entgegenhaltungen :
EP-A- 0 121 671
DE-A- 2 041 124
DE-A- 3 225 307
DE-B- 2 410 742
Patent Abstracts of Japan Band 4, Nr. 106, 30. Juli
1980 Seite 24C20

(73) Patentinhaber : Degussa Aktiengesellschaft
Weissfrauenstrasse 9
D-6000 Frankfurt am Main 1 (DE)

(72) Erfinder : Drauz, Karlheinz, Dr. Dipl.-Chem.
Flurstrasse 5
D-6463 Freigericht 1 (DE)
Erfinder : Kleemann, Axel, Dr. Dipl.-Chem.
Greifenhagenstrasse
D-6450 Hanau 9 (DE)

EP 0 121 672 B1

## Beschreibung

Die Erfindung betrifft die Herstellung von Brenzcatechin und Hydrochinon durch Kernhydroxylierung von Phenol mit Wasserstoffperoxid.

Brenzcatechin und Hydrochinon sind Abkömmlinge von Phenol. Sie lassen sich bei der Herstellung von Farbstoffen, in der Kunststoffproduktion, bei der Photographie oder zur Herstellung wichtiger Pflanzenschutzmittel einsetzen.

Ihre Herstellung, wie die der mehrwertigen Phenole überhaupt, war daher schon lange Gegenstand eingehender Untersuchungen. Die Hydroxylierung wurde sowohl mit Wasserstoffperoxid selbst, wie auch mit Hydroperoxiden, Peroxiden oder auch Persäuren, wie z. B. Perameisen- oder Peressigsäure, durchgeführt.

Doch war Wasserstoffperoxid, da es am leichtesten zugänglich war, bevorzugt, da mit Percarbonsäuren, Hydroperoxiden, Peroxiden Nebenreaktionen auftraten (EU-OS 0 027 593).

Stets war bei diesen Hydroxylierungen ein Katalysator anwesend. Dieser Katalysator konnte ein Metalloid, wie Schwefel, Selen, Tellur, Phosphor, Arsen oder Antimon in elementarer Form sein (DE-OS 2 348 957), oder man verwendete Borverbindungen (DE-PS 1 543 830).

Verschiedene Verfahren arbeiteten mit Übergangselementen in Form ihrer Ionen (DE-OS 2 162 552), besonders mit Eisenionen (DE-OS 2 162 589 oder DE-PS 2 407 398) oder Kobaltionen (DE-AS 2 341 743), oder auch mit den entsprechenden Oxiden (US-PS 2 395 638).

Ausserdem wurden starke Säuren, wie Schwefelsäure, Sulfonsäuren (DE-OS 2 138 735, DE-AS 2 410 742, DE-AS 2 410 758, DE-AS 2 462 967) oder Gemische aus Schwefel- und Phorphorsäure (DE-OS 2 138 735) eingesetzt, bzw. wurden in dieser letzgenannten Offenlegungschrift organische Säuren, wie u. a. Trichloressigsäure oder Weinsäure, genannt.

Die schon genannten Percarbonsäuren dienten ebenfalls als Katalysatoren (FR-PS 1 479 354). Bei den Katalysatoren handelte es sich in allen genannten Fällen um feste oder flüssige Substanzen. Wasserstoffperoxid — als bevorzugtes Oxydationsmittel — wurde meist in wässriger Lösung verschiedener Konzentrationen bis hin zu sehr hohen, explosionsgefährlichen Konzentrationen eingesetzt ; so arbeitete das Verfahren nach DE-PS 2 064 497 mit Lösungen, die nur noch 5 Gew.-% Wasser enthielten, aber selbst bei diesem höchstkonzentriertem Wasserstoffperoxid lag die Ausbeute an Dihydroxyderivaten nur bei 70 % und sank entsprechend der Verdünnung des Wasserstoffperoxide erheblich ab.

Hinzu kam, dass in diesem wie auch in anderen Verfahren mit einem sehr grossen Überschuss an dem zu hydroxylierenden Phenol gearbeitet werden musste, um überhaupt die oben angegebene Ausbeute zu erhalten. Wurde dieser Überschuss gesenkt, z. B. von 20 Mol auf 10 Mol pro Mol Wasserstoffperoxid, so sank trotz hoher Konzentration an Wasserstoffperoxid die Ausbeute drastisch.

Bekanntlich erfordern aber derartige Überschüsse an einer Reaktionskomponenten, die ja zurückgeführt werden muss, zusätzliche technische Aufwendungen ; vor allem hinsichtlich der Grösse der einzusetzenden Apparaturen.

Da man immer bemüht ist, grosse Überschüsse an einer Komponente nach Möglichkeit zu vermeiden, wurde versucht, den Einsatz wäßriger Lösungen von Wasserstoffperoxid zum umgehen.

So wurden schon verschiedentlich Lösungen von Wasserstoffperoxid in organischen Lösungsmitteln verwendet. Man arbeitete z. B. nach dem Verfahren der DE-PS 2 410 758 bevorzugt mit Wasserstoffperoxidlösungen in Abkömmlingen der Phosphor- oder Phosphonsäure, und zwar in Gegenwart einer starken Säure, wie Schwefelsäure (100 %ig) oder Fluorsulfonsäure.

Diese hochkonzentrierten starken Säuren haben aber den Nachteil, dass ihre Abtrennung aus dem Reaktionsgemisch Schwierigkeiten bereitet (DE-AS 2 658 943), vor allem, da ihre Konzentration im Reaktionsgemisch die Reaktionsdauer erheblich beeinflusst.

Die Überschüsse an Phenol waren zwar gegenüber denen im Verfahren der DE-AS 2 064 497 etwas vermindert, aber dies wog den Nachteil durch die starken Säuren nicht auf.

Eine zusätzliche Schwierigkeit beim Verfahren der DE-PS 2 410 758 bei der Aufarbeitung des Reaktionsgemisches wurde durch die Anwesenheit des nach der Reaktion mit Wasserstoffperoxid gebildeten Wassers hervorgerufen.

Da die eingesetzten Lösungsmittel für Wasserstoffperoxid zum Teil höher siedeten als das eingesetzte Phenol und dieses mit Wasser ein Azeotrop bildet, dessen Siedepunkt unter denen der organischen Lösungsmittel lag, war eine einwandfreie Abtrennung des überschüssigen Phenols aus dem Reaktionsgemisch äusserst problematisch.

Man ging daher andere Wege und versuchte, zunächst einmal ohne Katalysator, d. h. vor allem ohne die starken Säuren, auszukommen. Da die Katalysatoren in erster Linie für die Aktivierung von Wasserstoffperoxid notwendig waren, wurde im Verfahren der DE-AS 26 58 843 mit organischen Lösungen von Percarbonsäuren gearbeitet. Ein zusätzlicher Katalysator wurde nicht verwendet.

Ganz abgesehen davon, dass das genannte Verfahren eine vollständige Anlage zur Herstellung einer organischen Percarbonsäure, die zunächst aus Wasserstoffperoxid und Carbonsäure gewonnen und darauf durch Extraktion dieser sog. « Gleichgewichtssäure » aus ihrem wäßrigen Medium hergestellt wird, voraussetzt, hatte sich gezeigt, dass eine angeblich gute Selektivität und gute Ausbeute nur durch Anwesenheit zusätzlicher Persäurestabilisatoren möglich war (DE-OS 23 64 181 ; EU-OS 0 027 593).

Auch der Versuch, Brenzcatechin und Hydrochinon ohne Katalysator mit dampfförmigem Wasserstoffperoxid herzustellen, war wegen der Explosionsgefährlichkeit schlecht technisch durchführbar (JP-OS 24056/1974).

Nach dem Obengesagten ergibt sich, dass Verfahren, die Wasserstoffperoxid als das einfachste und am besten zugängliche Hydroxylierungsmittel verwendeten, bei der technischen Herstellung von Dihydroxybenzolen keine insgesamt befriedigende Verfahrensweise ermöglichten.

Daher wurden in neuerer Zeit nur Verfahren entwickelt, die nicht direkt Wasserstoffperoxid verwendeten und aus dem Grund teilweise hohe technische Aufwendungen notwendig machten.

Ziel der Erfindung ist es daher, die Kernhydroxylierung von Phenol mit Wasserstoffperoxid in Gegenwart von Katalysatoren in technisch einfacher Weise und sehr guten Ausbeuten durchzuführen.

Es wurde nun gefunden, daß sich diese Aufgabe lösen läßt bei Verwendung von organischen Lösungen von Wasserstoffperoxid, wenn man die Umsetzung mit Lösungen von Wasserstoffperoxid, die wasserfrei sind und einen Wassergehalt von 0-1 Gew.-% haben und die mit organischen Lösungsmitteln hergestellt werden, die mit Wasser Azeotrope bilden, deren Azeotropsiedepunkte unter dem Siedepunkt von Wasserstoffperoxid, bezogen auf Normaldruck, liegen, sowie in Gegenwart von Schwefeldioxid in den Mengen von 0,000 1 bis 0,1 Mol, bezogen auf 1 Mol Wasserstoffperoxid, bei 20-200 °C im Molverhältnis von Phenol zu Wasserstoffperoxid von 5 bis 20 : 1, durchführt.

Als Lösungsmittel kommen auch in Frage Äther, wie Dioxan, Diisopropyläther, Methyl-tert.-butyläther.

Bevorzugte Lösungsmittel sind Alkyl- oder Cycloalkylester von gesättigten, aliphatischen Carbonsäuren, die eine Gesamtkohlenstoffzahl von 4-8 besitzen.

Besonders geeignete Ester sind die der Essig- oder Propionsäure, vor allem Essigsäure-n- oder -i-propylester.

Es können auch Gemische der Ester eingesetzt werden.

Die organischen Wasserstoffperoxidlösungen können in üblicher Form stabilisiert sein, siehe ULLMANN, Enzyklopädie der technischen Chemie, 4. Aufl., Band 17, Seite 709.

Die genannten Lösungen von Wasserstoffperoxid in Alkyl- oder Cycloalkylestern werden nach dem Verfahren der DE-A-32 25 307 gewonnen.

Das als Katalysator wirkende Schwefeldioxid kann in gasförmigem Zustand verwendet werden. Schwefeldioxid kann aber auch in einem beliebigen Lösungsmittel gelöst sein, das sowohl mit Schwefeldioxid selbst wie auch mit Wasserstoffperoxid keine störenden Reaktionen eingeht, beispielsweise in Dialkyläthern, Estern der Phosphor- oder Phosphonsäure. Die Konzentrationen richten sich nach der Löslichkeit vob SO$_2$ im Lösungsmittel, im allgemeinen liegen sie bei 0,1 bis 50, bevorzugt bei 1 bis 10 Gew.-%. Günstig ist es aber, Schwefeldioxid als Lösung in einem der oben beschriebenen Carbonsäureestern einzusetzen. Schwefeldioxid wird erfindungsgemäß in sehr geringen Mengen verwendet (siehe Anspruch 1), die bevorzugt bei 0,000 5 bis 0,01 Mol, bezogen auf 1 Mol Wasserstoffperoxid, liegen. Diese Mengen sind sehr gering, verglichen mit denen der Protonensäuren, die bei sauer katalysierten Hydroxylierungen verwendet werden.

Die Umsetzung findet bevorzugt bei Temperaturen von 40 bis 180 °C statt.

Die organischen Lösungen von Wasserstoffperoxid in den genannten Alkyl- oder Cycloalkylestern ermöglichen höhere Konzentrationen (bis über 60 Gew.-%) und zeichnen sich durch sehr geringe Wassergehalte (unter 0,5 Gew.-%) aus, siehe DE-A-32 25 307.

Das erfindungsgemäße Verfahren läßt sich — wie gesagt — für die Kernhydroxylierung von Phenol durchführen.

Der Druck ist für die Umsetzung nicht entscheidend ; sie wird im allgemeinen bei Normaldruck ausgeführt.

Die Reaktionsdauer hängt ab von der Temperatur und der Konzentration an Schwefeldioxid.

Um die beste Reaktionszeit festzustellen, kann man einen Handversuch durchführen.

Vorzugsweise wird so verfahren, dass nach 15 Minuten bereits mehr als 95 % des eingesetzten Wasserstoffperoxids umgesetzt sind.

Die Hydroxylierung von Phenol nach dem erfindungsgemässen Verfahren gelingt dann besonders gut, wenn wasserfreie Lösungen von Wasserstoffperoxid in den genannten Carbonsäureestern mit einem Gewichtsverhältnis von etwa 1 : 4 bis 2 : 1 H$_2$O$_2$/Carbonsäureester eingesetzt werden.

Dieses Gewichtsverhältnis erreicht man auch bei Verwendung von niedriger konzentrierten H$_2$O$_2$-Lösungen, indem man aus diesen Lösungen im Gemisch mit dem Phenol den Carbonsäureester über Kopf abdestilliert. Die Entnahme kann auf jeden beliebigen Wert einreguliert werden.

Bei der Destillation wird so verfahren, dass praktisch kein Phenol und kein Wasserstoffperoxid mit ausgetragen werden.

Als positiver Nebeneffekt findet noch eine azeotrope Entwässerung des Phenols statt, da das Azeotrop Wasser/Carbonsäureester die niedrigst siedende Komponente ist.

Die Aufbereitung des Reaktionsgemisches ist wesentlich einfacher als es bisher bekannt war.

Da die erfindungsgemäß als Lösungsmittel zu verwendenden Ester niedriger als die zur Umsetzung kommenden Phenole sieden, wird als erstes ein Azeotrop zwischen dem Ester und Wasser abdestilliert. Die Schwierigkeiten einer Wasser-Phenoltrennung, wie sie bisher aufgetreten sind, fallen fort. Dies ist besonders wichtig, da Phenol im Überschuß angewendet wird und wieder zurückgeführt werden muß.

Bei der Aufarbeitung ist es nicht unbedingt notwendig, infolge der extrem niedrigen Katalysatorkonzentrationen, vor einer destillativen Trennung eine Abtrennung des Katalysators, beispielsweise durch Neutralisation, vorzunehmen ; das rohe Reaktionsgemisch wird direkt einer Destillation unterworfen.

Das Molverhältnis von Phenol und Wasserstoffperoxid liegt bevorzugt bei 5 bis 15 : 1, ganz besonders günstig bei 7 bis 15 : 1.

Beim erfindungsgemäßen Verfahren wird zwar ein Katalysator eingesetzt, aber in solch geringen Mengen, daß seine gesonderte Abtrennung vor der destillativen Aufarbeitung des Reaktionsgemisches überflüssig wird.

Hinzu kommt nun, daß eine sehr günstige Raum-Zeit-Ausbeute aufgrund der kurzen Reaktionszeiten erzielt wird ; für die technische Durchführung genügen also kleine Reaktionsvolumina. So liegen 99 %ige Umsätze und mehr schon nach 20 bis 30 Minuten vor.

Durch die kurze Reaktionsdauer ist außerdem gleichzeitig die mögliche Gefahr von Zersetzungen vermindert. Diese Reaktion läßt sich auch gut kontinuierlich durchführen.

Wesentlich ist ferner, daß die leichtsiedenden Lösungsmittel gemeinsam mit dem vorhandenen Wasser einwandfrei von dem restlichen Phenol und den Reaktionsprodukten abgetrennt werden können, so daß das Phenol praktisch wasserfrei wieder in die Umsetzungsstufe zurückgeführt werden kann.

Alle diese Vorteile sind nicht mit einer Verringerung der bisher nach dem Stand der Technik erhaltenen Ausbeuten verbunden, sondern die Ausbeuten selbst sind erhöht.

Die Erfindung wird anhand nachstehender Beispiele näher erläutert.

Beispiel 1

94,1 g (1,0 mol) Phenol werden auf 100 °C erwärmt. Zu der gerührten Schmelze setzt man 0,4 g einer 4,8 Gew.-%igen Lösung von Schwefeldioxid in Essigsäure-n-propylester zu und anschließend 6,37 g einer 53,4 Gew.-%igen wasserfreien Lösung von Wasserstoffperoxid in Essigsäure-n-propylester (= 0,1 mol). Die Temperatur in der Reaktionslösung erhöht sich danach auf 154 °C. Nach Abklingen der Exotherme wird nach 10 min. ein Wasserstoffperoxidumsatz von 98,3 % bestimmt. Das Reaktionsgemisch enthält dann 3,15 g (28,6 mMol) Hydrochinon und 6,47 g (58,7 mMol) Brenzkatechin, was einer Gesamtausbeute an Dihydroxybenzolen von 88,9 %, bezogen auf umgesetztes Wasserstoffperoxid, entspricht.

Beispiel 2

141 g (1,5 mol) Phenol werden auf 100 °C erwärmt. Zu der gerührten Schmelze setzt man 0,4 g einer 4,8 Gew.-%igen Lösung von Schwefeldioxid in Essigsäure-n-propylester zu und anschließend 6,37 g einer 53,4 Gew.-%igen wasserfreien Lösung von Wasserstoffperoxid in Essigsäure-n-propylester (0,1 mol). Die Temperatur in der Reaktionslösung steigt danach auf 126 °C. Nach Abklingen der Exotherme wird nach 20 min. ein Wasserstoffperoxidumsatz von 90,4 % ermittelt. Das Reaktionsgemisch enthält dann 3,05 g (27,7 mMol) Hydrochinon und 6,15 g (55,8 mMol) Brenzkatechin, was einer Gesamtausbeute an Dihydroxybenzolen von 92,4 %, bezogen auf umgesetztes $H_2O_2$, entspricht.

Beispiel 3

65,9 g (0,7 mol) Phenol werden auf 100 °C erwärmt. Zu der gerührten Schmelze setzt man 0,27 g einer 4,8 Gew.-%igen Lösung von Schwefeldioxid in Essigsäure-n-propylester zu und anschließend 6,37 g einer 53,4 Gew.-%igen wasserfreien Lösung von Wasserstoffperoxid in Essigsäure-n-propylester (0,1 mol). Die Temperatur in der Reaktionsmischung steigt danach auf 152 °C. Nach Abklingen der Exotherme wird nach 5 min. ein Wasserstoffperoxidumsatz von 91,4 % festgestellt. Das Reaktionsgemisch enthält dann 2,86 g (25,9 mMol) Hydrochinon und 5,35 g (48,6 mMol) Brenzkatechin, was einer Gesamtausbeute an Dihydroxybenzolen von 81,6 %, bezogen auf umgesetztes Wasserstoffperoxid, entspricht.

Beispiel 4

94,1 g (1,0 mol) Phenol werden auf 100 °C erwärmt. Zu der gerührten Schmelze setzt man 0,4 g einer 4,8 Gew.-%igen Lösung von Schwefeldioxid in Essigsäure-n-propylester zu und anschließend 5,4 g einer 63 Gew.-%igen wasserfreien Lösung von Wasserstoffperoxid (0,1 mol) in Essigsäureethylester. Die Temperatur steigt auf 152 °C an. Nach Abklingen der Exotherme wird nach 5 min. ein Wasserstoffperoxidumsatz von 96,7 % ermittelt. Das Reaktionsgemisch enthält dann 2,82 g (25,6 mMol) Hydrochinon und 5,87 g (53,3 mMol) Brenzkatechin, was einer Gesamtausbeute an Dihydroxybenzolen von 81,6 %, bezogen auf umgesetztes Wasserstoffperoxid, entspricht.

Beispiel 5

94,1 g (1,0 mol) Phenol werden auf 100 °C erwärmt. Zu der gerührten Schmelze setzt man 0,4 g einer 4,8 Gew.-%igen Lösung von Schwefeldioxid in Essigsäure-n-propylester und anschließend 5,7 g einer 59,6 Gew.-%igen wasserfreien Lösung von Wasserstoffperoxid (0,1 mol) in Propionsäuremethylester zu. Die Temperatur steigt auf 151 °C. Nach Abklingen der Exotherme wird nach 10 min. ein Wasserstoffperoxidumsatz von 98,5 % festgestellt. Das Reaktionsgemisch enthält dann 2,95 g (26,8 mMol) Hydrochinon und 6,15 g (55,8 mMol) Brenzkatechin, was einer Gesamtausbeute an Dihydroxybenzolen von 83,9 %, bezogen auf umgesetztes Wasserstoffperoxid, entspricht.

Beispiel 6

94,1 g (1,0 mol) Phenol werden auf 100 °C erwärmt. Zu der gerührten Schmelze setzt man 0,4 g einer 4,8 Gew.-%igen Lösung von Schwefeldioxid in Essigsäure-n-propylester zu und anschließend 11,5 g einer 29,5 Gew-%igen wasserfreien Lösung von Wasserstoffperoxid in Propionsäuremethylester (0,1 mol). Die Temperatur steigt auf 146 °C. Nach Abklingen der Exotherme wird nach 5 min. ein Wasserstoffperoxidumsatz von 95,7 % ermittelt. Das Reaktionsgemisch enthält dann 2,94 g (26,7 mMol) Hydrochinon und 5,97 g (54,2 mMol) Brenzkatechin, was einer Gesamtausbeute an Dihydroxybenzolen von 84,5 %, bezogen auf umgesetztes Wasserstoffperoxid, entspricht.

Beispiel 7

94,1 g (1,0 mol) Phenol werden auf 100 °C erwärmt. Zu der gerührten Schmelze setzt man 0,4 g einer 4,8 Gew.-%igen Lösung von Schwefeldioxid in Essigsäure-n-propylester zu und anschließend 7,4 g einer 46,1 Gew.-%igen wasserfreien Lösung von Wasserstoffperoxid in Propionsäureethylester (0,1 mol) zu. Die Temperatur steigt auf 153 °C. Nach Abklingen der Exotherme wird nach 20 min. ein Wasserstoffperoxidumsatz von 99,4 % ermittelt. Das Reaktionsgemisch enthält dann 2,65 g (24,1 mMol) Hydrochinon und 5,63 g (51,1 mMol) Brenzkatechin, was einer Gesamtausbeute von 75,6 %, bezogen auf umgesetztes Wasserstoffperoxid, entspricht.

Beispiel 8

Vergleichsbeispiel zu Beispiel 2 ; wurde nicht nach dem erfindungsgemäßen Verfahren hergestellt :

141,1 g (1,5 mol) Phenol werden auf 110 °C erwärmt. Zu der gerührten Schmelze setzt man 0,02 g einer 100 %-igen Schwefelsäure und anschließend 6,37 g einer 53,4 Gew.-%igen wasserfreien Lösung von Wasserstoffperoxid in Essigsäurepropylester (0,1 mol). Die Temperatur steigt auf 135 °C. Nach Abklingen der Exotherme wird nach 60 min. ein Wasserstoffperoxidumsatz von 99,4 % ermittelt. Das Reaktionsgemisch enthält 1,61 g (14,6 mMol) Hydrochinon und 4,34 g (39,4 mMol) Brenzkatechin, was einer Gesamtausbeute an Dihydroxybenzolen von 54,4 %, bezogen auf Wasserstoffperoxid, entspricht.

## Patentansprüche

1. Verfahren zur Herstellung von Brenzcatechin und Hydrochinon durch Kernhydroxylierung von Phenol mit Wasserstoffperoxid in Form einer wasserfreien organischen Lösung in Gegenwart eines Katalysators, dadurch gekennzeichnet, daß man die Umsetzung mit Lösungen von Wasserstoffperoxid, die wasserfrei sind und einen Wassergehalt von 0-1 Gew.-% haben und die mit organischen Lösungsmitteln hergestellt werden, die mit Wasser Azeotrope bilden, deren Azeotropsiedepunkte unter dem Siedepunkt von Wasserstoffperoxid, bezogen auf Normaldruck, liegen, sowie in Gegenwart von Schwefeldioxid in den Mengen von 0,000 1-0,1 Mol, bezogen auf 1 Mol Wasserstoffperoxid, bei 20-200 °C im Molverhältnis von Phenol zu Wasserstoffperoxid von 5 bis 20 : 1, durchführt.

2. Verfahren nach Anspruch 1, dadurch gekennzeichnet, daß man Wasserstoffperoxidlösungen in Alkyl- oder Cycloalkylestern von gesättigten, aliphatischen Carbonsäuren, die eine Gesamtkohlenstoffzahl von 4-8 besitzen, einsetzt.

3. Verfahren nach Anspruch 1 und 2, dadurch gekennzeichnet, daß man Wasserstoffperoxidlösungen von Estern der Essig- oder Propionsäure verwendet.

4. Verfahren nach Anspruch 1 bis 3, dadurch gekennzeichnet, daß man Wasserstoffperoxidlösungen in Essigsäure-n-propylester oder Essigsäure-i-propylester verwendet.

5. Verfahren nach Anspruch 1 bis 4, dadurch gekennzeichnet, daß man das Schwefeldioxid in Form einer Lösung der in Anspruch 2 genannten Alkyl- oder Cycloalkylester einsetzt.

6. Verfahren nach Anspruch 1, dadurch gekennzeichnet, daß man $SO_2$ in gasförmigem Zustand einsetzt.

7. Verfahren nach Anspruch 1 bis 6, dadurch gekennzeichnet, daß man Schwefeldioxid in Mengen von 0,000 5 bis 0,01 Mol, bezogen auf 1 Mol Wasserstoffperoxid, einsetzt.

8. Verfahren nach Anspruch 1 bis 7, dadurch gekennzeichnet, daß man ein Molverhältnis von Phenol zu Wasserstoffperoxid von 5 bis 15 : 1, besonders bevorzugt von 7 bis 15 : 1, einsetzt.

9. Verfahren nach Anspruch 1 bis 8, dadurch gekennzeichnet, daß man bei Temperaturen von 40-180 °C arbeitet.

## Claims

1. A process for the production of pyrocatechol and hydroquinone by nuclear hydroxylation of phenol with hydrogen peroxide in the form of an anhydrous organic solution in the presence of a catalyst, characterised in that the reaction is carried out with solutions of hydrogen peroxide which are anhydrous and have a water-content of from 0 to 1 % by weight and which are produced with organic solvents which form with water azeotropes whose azeotropic boiling points lie below the boiling point of hydrogen peroxide, with respect to normal pressure, and in the presence of sulphur dioxide in quantities of from 0.000 1 to 0.1 mol, based on 1 mol of hydrogen peroxide, at from 20 to 200 °C in a phenol to hydrogen peroxide molar ratio of from 5 to 20 : 1.

2. A process according to Claim 1, characterised in that hydrogen peroxide solutions in alkyl or cycloalkyl esters of saturated aliphatic carboxylic acids having a total carbon number of from 4 to 8 are used.

3. A process according to Claims 1 and 2, characterised in that hydrogen peroxide solutions of esters of acetic or propionic acid are used.

4. A process according to Claims 1 to 3, characterised in that hydrogen peroxide solutions in acetic acid n-propylester or acetic acid i-propylester are used.

5. A process according to Claims 1 to 4, characterised in that the sulphur dioxide is used in the form of a solution of the alkyl or cycloalkyl ester mentioned in Claim 2.

6. A process according to Claim 1, characterised in that $SO_2$ is used in the gaseous state.

7. A process according to Claims 1 to 6, characterised in that sulphur dioxide is used in quantities of from 0.000 5 to 0.01 mol, based on 1 mol of hydrogen peroxide.

8. A process according to Claims 1 to 7, characterised in that a phenol to hydrogen peroxide molar ratio of from 5 to 15 : 1, particularly preferably from 7 to 15 : 1 is used.

9. A process according to Claims 1 to 8, characterised in that it is carried out at temperatures of from 40 to 180 °C.

**Revendications**

1. Procédé pour la fabrication de pyrocatéchine et d'hydroquinone par hydroxylation sur le noyau du phénol avec du peroxyde d'hydrogène sous forme d'une solution organique anhydre, en présence d'un catalyseur, caractérisé en ce que l'on réalise la réaction avec des solutions de peroxyde d'hydrogène qui sont anhydres et qui ont une teneur en eau de 0 à 1 % en poids, et qui sont préparées avec des solvants organiques, qui forment avec l'eau des azéotropes, le point d'ébullition de ces azéotropes étant inférieur à celui du peroxyde d'hydrogène, considéré à la pression normale, ainsi qu'en présence d'anhydride sulfureux dans des proportions de 0,000 1 à 0,1 mole, calculé sur une mole de peroxyde d'hydrogène, la réaction s'effectuant de 20 à 200 °C, dans le rapport moléculaire du phénol au peroxyde d'hydrogène de 5 à 20 : 1.

2. Procédé suivant la revendication 1, caractérisé en ce qu'il est utilisé des solutions du peroxyde d'hydrogène dans des esters alcoyliques ou cycloalcoyliques d'acides carboxyliques aliphatiques saturés, qui possèdent un nombre total d'atomes de carbone de 4 à 8.

3. Procédé suivant les revendications 1 et 2, caractérisé en ce qu'il est utilisé des solutions de peroxyde d'hydrogène dans des esters des acides acétique ou proprionique.

4. Procédé suivant les revendications 1 à 3, caractérisé en ce qu'il est utilisé des solutions de peroxyde d'hydrogène dans des esters n-propylique ou i-propylique de l'acide acétique.

5. Procédé suivant les revendications 1 à 4, caractérisé en ce qu'il est utilisé de l'anhydride sulfureux sous la forme d'une solution dans les esters alcoyliques ou cycloalcoyliques mentionnés dans la revendication 2.

6. Procédé suivant la revendication 1, caractérisé en ce qu'il est utilisé $SO_2$ à l'état gazeux.

7. Procédé suivant les revendications 1 à 6, caractérisé en ce qu'il est utilisé de l'anhydride sulfureux dans des proportions de 0,000 5 à 0,01 mole, calculé par mole de peroxyde d'hydrogène.

8. Procédé suivant les revendications 1 à 7, caractérisé en ce que l'on met en œuvre un rapport moléculaire du phénol au peroxyde d'hydrogène de 5 à 15 : 1, et particulièrement recommandé, de 7 à 15 : 1.

9. Procédé suivant l'une des revendications 1 à 8, caractérisé en ce que l'on opère à des températures de 40 à 180 °C.